Europäisches Patentamt

European Patent Office

Office européen des brevets

(19)

(11) Veröffentlichungsnummer: **0 295 360 B1**

(12) **EUROPÄISCHE PATENTSCHRIFT**

(45) Veröffentlichungstag der Patentschrift:
13.03.91 Patentblatt 91/11

(51) Int. Cl.⁵: **A61F 2/36**

(21) Anmeldenummer: **88100625.8**

(22) Anmeldetag: **18.01.88**

(54) **Endoprothese.**

(30) Priorität: **15.06.87 DE 8708419 U**

(43) Veröffentlichungstag der Anmeldung:
**21.12.88 Patentblatt 88/51**

(45) Bekanntmachung des Hinweises auf die
Patenterteilung:
**13.03.91 Patentblatt 91/11**

(84) Benannte Vertragsstaaten:
**AT CH DE ES FR GB IT LI NL SE**

(56) Entgegenhaltungen:
**EP-A- 0 158 014
DE-A- 2 305 333
DE-U- 8 212 788**

(73) Patentinhaber: **Waldemar Link GmbH & Co
Barkhausenweg 10
W-2000 Hamburg 63 (DE)**

(72) Erfinder: **Link, Helmut D.
Wildstieg 14
W-2000 Hamburg 65 (DE)**
Erfinder: **Keller, Arnold
An der Naherfurth 5
W-2061 Kayhude (DE)**

(74) Vertreter: **Glawe, Delfs, Moll & Partner
Patentanwälte
Postfach 162 Liebherrstrasse 20
W-8000 München 26 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf eine Endoprothese zum Ersatz eines Knochenteils, insbesondere Hüftgelenkprothese, die einen in einer Knochenhöhlung zu verankernden Schaft und eine sich quer zum Schaft erstreckende, gesonderte Knochenauflage umfaßt, die durch zusammenwirkende, an der Knochenauflage und am oberen Schaftende angeordnete Halteeinrichtungen in Längsrichtung des Schattes gehalten ist. Eine derartige Endoprothese ist beispielsweise ans der EP-A- 0158 014 bekannt.

Man strebt, insbesondere bei zementloser Implantation, eine genaue Anlage der Knochenauflage der Prothese auf der entsprechenden Knochenfläche an. Das gilt insbesondere für die Knochenauflage einer Hüftgelenkendoprothese, die einen wesentlichen Teil der Körperlast auf die Resektionsfläche des Oberschenkelknochens überträgt. Weil der den Hals abtrennende Resektionsschnitt für eine vollflächige Anlage der Knochenauflage oftmals nicht genau genug ist, steht dem Arzt ein besonderes Instrumentarium zur planparallelen Nachresektion des Oberschenkelknochens zur Verfügung. Dazu gehört ein Schenkelhalsfräser, der über eine in die ausgeraspelte Markhöhle des Oberschenkelknochens eingesetzte Lehre so geführt wird, daß alle Winkel der Resektionsflächen mit den Winkeln der Knochenauflage übereinstimmen und ein formschlüssiger Aufsitz der Knochenauflage auf dem Knochen erreicht wird. Diese Nachresektion verlängert die Operationsdauer und ist deshalb in vielen Fällen, insbesondere bei älteren Menschen, unerwünscht.

Es ist bekannt (EP-A 0 158 014 ; DE-A 23 05 333), zur Vermeidung der Nachresektion eine Vielzahl alternativ verwendbarer Knochenauflageplatten zu verwenden, die einerseits winkelfest mit dem Prothesenschaft verbunden werden können, aber andererseits eine unter verschiedenen Winkeln geneigte Unterfläche zur Anpassung an unterschiedliche Winkellage der Resektionsfläche aufweisen. Ihre Verwendung setzt voraus, daß die Winkellage der Resektionsfläche im Verhältnis zur Prothesenrichtung genau gemessen wird, was unter Operationsbedingungen oftmals schwierig ist und zu fehlerhafter Auswahl führen kann. Überdies können die auswechselbaren Knochenauflagen aus Kostengründen nur in verhältnismäßig groben Winkelabstufungen zur Verfügung gestellt werden, so daß sie gewisse Winkelabweichungen zwischen der Knochenauflagefläche der Prothese und der Resektionsfläche nicht ausschließen.

Der Erfindung liegt die Aufgabe zugrunde, einen guten Paßsitz der Prothese zu erreichen und dennoch die Notwendigkeit der Nachresektion zu vermeiden.

Die erfindungsgemäße Lösung besteht darin, daß die die Knochenauflage am oberen Schaftende sichernden Halteeinrichtungen eneinander schwenkbeweglich ausgebildet sind, so daß die : Unochenanflage in ihrer Winkellage zur Schaftachse veränderbar ist. Die Knochenauflage nichtet sich somit selbsttätig auf die zugehörige Knochenfläche aus.

In manchen Fällen mag eine Schwenkbeweglichkeit um eine Achse ausreichen ; im allgemeinen wird jedoch eine allseitige Schwenkbeweglichkeit bevorzugt. Dafür eignen sich Halteeinrichtungen, die schaftseitig von einem Ringvorsprung mit konvexsphärischer Unterfläche und auf seiten der Knochenauflage von einer entsprechenden Gegenfläche gebildet sind, wobei letztere zweckmäßigerweise eine sphärische Ausnehmung ist.

Die Knochenauflage ist zweckmäßigerweise hufeisenförmig ausgebildet und von der Seite her auf den Schaft aufsteckbar, wie dies an sich bekannt ist (EP-PS 0 093 230 ; DE-U 82 12 788).

Die Erfindung wird im folgenden näher unter Bezugnahme auf die Zeichnung erläutert, die ein vorteilhaftes Ausführungsbeispiel einer Hüftgelenkprothese veranschaulicht. Darin zeigen :

Fig. 1 und 2 zwei Seitenansichten des oberen Teils,

Fig. 3 und 4 einen Längschnitt und eine Draufsicht der Knochenauflage,

Fig. 5 und 6 Seitenansichten des mit der Knochenauflage verbundenen Schafts.

Der im Oberschenkelknochen zu verankernde Schaftteil 1, der in beliebiger, herkömmlicher Weise gestaltet sein kann und dessen unterer Teil in der Zeichnung nicht veranschaulicht ist, trägt einen Trochantersporn 2 und endet in der oberen Stirnfläche 3, von der der Halsteil 4 ausgeht. Der obere Abschnitt 5 des Halsteils ist konisch ausgebildet zur Aufnahme des Gelenkkopfs. Er trägt ferner einen Ringvorsprung 6, dessen Unterfläche 7 sphärisch konvex ausgebildet ist. Zwischen dieser Fläche 7 und der oberen Stirnfläche 3 des Schaftteils 1 bildet der Hals eine nutartige Verengung 8.

Die Knochenauflage 9 besitzt eine ebene Unterfläche 10, die dazu bestimmt ist, sich auf der Resektionsfläche des Oberschenkelknochens abzustützen. Die Oberseite enthält eine konkave, sphärische Ausnehmung 11, die im wesentlichen denselben Krümmungsradius wie die sphärische Unterfläche 7 des Ringvorsprungs 6 besitzt. Die Knochenauflage 9 ist hufeisenförmig gestaltet mit einem Ausschnitt, dessen innerer Teil 12 kreisförmig begrenzt ist, konzentrisch zu der Ausnehmung 11, während ihr äußerer Teil 13, dessen Breite dem inneren Teil 12 gleicht, sich zwischen parallelen Begrenzungen 14 nach außen öffnet. Betrachtet man die Begrenzungsflächen 14, so stellt man fest, daß ihre Höhe im inneren Bereich durch die sphärische Ringausnehmung 11 verringert ist, während sie nahe dem Außenumfang der Knochenauflage im Bereich 15 erhöht ist.

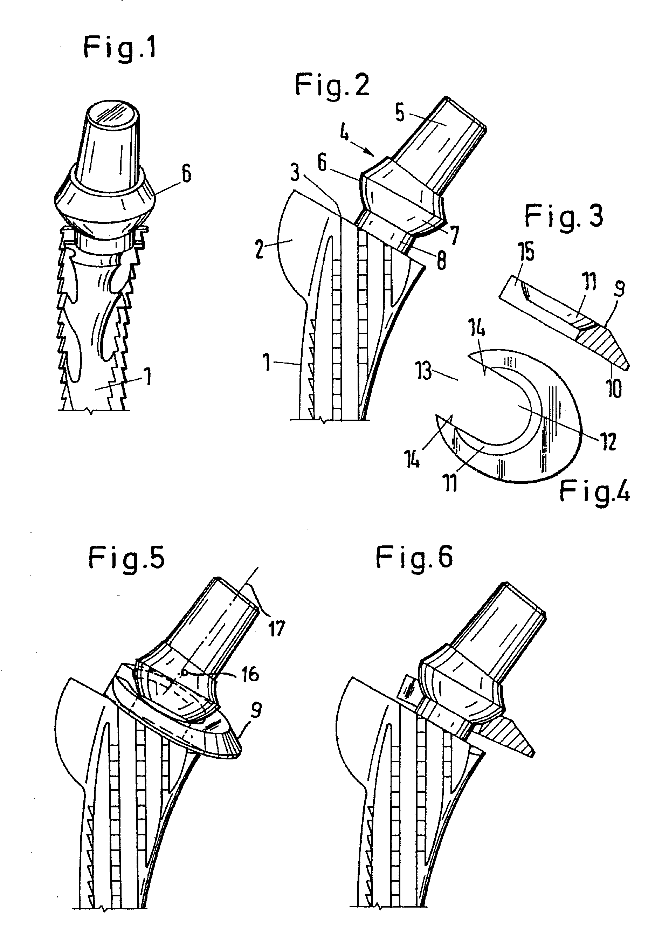

Die Abmessungen sind so gewählt, daß die Knochenauflage 9 im Bereich der Verengung 8 auf den Hals des Prothesenschafts aufgeschoben werden kann, wie dies in Fig. 5 angedeutet ist. Bei der Implantation der Prothese legt sich die Unterfläche 10 auf die Resektionsfläche des Oberschenkelknochens, wobei sich die sphärische Fläche 7 des Ringvorsprungs 6, wie in Fig. 6 dargestellt, in die entsprechend sphärische Ausnehmung 11 der Knochenauflage legt und sich daran abstützt. Infolge der sphärischen Gestalt dieser Teile kann die Knochenauflage sich frei im Winkel einstellen und sich daher auch dann vollflächig auf die Resektionsfläche auflegen, wenn diese nicht absolut rechtwinklig zu dem zugehörigen Teil des Prothesenschafts stehen sollte. Vollflächige Auflage und günstigste Kraftübertragung ist auch dann gewährleistet, wenn sich im Laufe der Zeit eine Verlagerung der Prothese im Knochen oder eine Änderung der Knochenform ergeben sollte.

Die schwenk- bzw. kugelbeweglichen Halterungen, die zwischen der Knochenauflage und dem Schaft wirken, können beliebig ausgestaltet sein. Der Krümmungsmittelpunkt 16 der sphärischen Fläche 7 liegt bei einer bevorzugten Ausführungsform, die in der Zeichnung dargestellt ist, nahe der Mittelachse 17 des Halsteils 4. Vorteilhaft kann es auch sein, wenn der Mittelpunkt medial von der Achse 17 liegt.

Wesentlich ist, daß sich die Knochenauflage beim Eintreiben des Prothesenschafts selbsttätig auf die möglicherweise schräg angelegte Resektionsfläche des Oberschenkelknochens formschlüssig auflegt. Bei der dargestellten Ausführung muß zwischen der Unterfläche 10 der Knochenauflage und der oberen Stirnfläche 3 des Schaftteils 1 so viel Luft vorhanden sein, daß sich die Knochenauflage auch schräg ausrichten kann, wenn dies durch die Lage der Resektionsebene vorgegeben ist.

Die Abnehmbarkeit der Knochenauflage ist keine Vorbedingungen für die Schwenkbeweglichkeit der Knochenauflage. Vielmehr kann die Knochenauflage fabrikseitig unlösbar, wenn auch beweglich, am Prothesenschaft montiert sein.

Wenngleich die Vorteile der Erfindung bei Hüftgelenkprothesen besonders augenfällig sind, ist sie auch bei anderen Prothesen anwendbar, bei denen ein Teil der Kraft von der Prothese über eine Knochenauflage auf eine resezierte oder natürlich vorhandene Fläche des zugehörigen Knochens übertragen wird, beispielsweise Schulterprothesen, Ellenbogengelenkprothesen, Kniegelenkprothesen, Fingergelenkprothesen. Die Knochenauflage, die ein vom Schaft gesonderter Teil ist, kann mit anderen Prothesenteilen fest verbunden sein, beispielsweise mit einem gelenkbildenden Teil.

Knochenhöhlung zu verankernden Schaft (1) und eine sich quer zum Schaft erstreckende gesonderte Knochenauflage (9) umfaßt, die durch zusammenwirkende, an der Knochenauflage (9) und am oberen Schaftende angeordnete Halteeinrichtungen (7, 11) in Längsrichtung des Schaftes (1) gehalten ist, dadurch gekennzeichnet, daß die Malteeinrichtungen (7, 11) zueinander schwenkbeweglich ausgebildet sind, so daß die Knochenanflage (9) in ihrer Winkellage zur Schaftachse veränderbar ist.

2. Endoprothese nach Anspruch 1, dadurch gekennzeichnet, daß die Halteeinrichtungen allseitig schwenkbeweglich ausgebildet sind.

3. Endoprothese nach Anspruch 2, dadurch gekennzeichnet, daß die Halteeinrichtungen schaftseitig von einem Ringvorsprung (6) mit konvex-sphärischer Unterfläche (7) und auf seiten der Knochenauflage von einer entsprechenden Ausnehmung (11) gebildet sind.

4. Endoprothese nach einem der Ansprüche 1 bis 3, dadurch gekennzeichnet, daß die Knochenauflage (9) hufeisenförmig ausgebildet und von der Seite her auf den Schaft aufsteckbar ist.

## Claims

1. An endoprosthesis for the replacement of a bone portion, in particular a hip-joint prosthesis, which comprises a shaft (1) to be anchored in a bone cavity and a separate bone support (9) which extends transversely to the shaft and which is retained in the longitudinal direction of the shaft (1) by co-operating retaining means (7, 11) mounted on the bone support (9) and on the upper shaft end, characterised in that the retaining means (7, 11) are formed so as to be pivotally movable relative to one another, so that the bone support (9) is variable in its angular position relative to the shaft axis.

2. An endoprosthesis according to Claim 1, characterised in that the retaining means are formed so as to be pivotally movable on all sides.

3. An endoprosthesis according to Claim 2, characterised in that on the shaft side the retaining means are formed by an annular projection (6) with a convex spherical underside (7) and on the bone support side they are formed by a corresponding recess (11).

4. An endoprosthesis according to any one of Claims 1 to 3, characterised in that the bone support (9) is horseshoe-shaped and can be fitted on to the shaft from the side.

## Ansprüche

1. Endoprothese zum Ersatz eines Knochenteils, insbesondere Hüftgelenkprothese, die einen in einer

## Revendications

1. Endoprothèse pour le remplacement d'un fragment osseux, en particulier prothèse de hanche, comprenant une tige (1) pour son ancrage dans une cavité osseuse et une pièce séparée (9) d'appui sur l'os qui s'étend transversalement par rapport à la tige et qui est maintenue dans la direction longitudinale de la tige (1) par des moyens de retenue coopérants (7, 11) qui sont disposés respectivement sur la pièce (9) d'appui sur l'os et à l'extrémité supérieure de la tige, caractérisée en ce que les moyens de retenue (7, 11) sont réalisés de manière à pouvoir pivoter l'un par rapport à l'autre, de telle sorte que la position angulaire de la pièce (9) d'appui sur l'os par rapport à l'axe de la tige puisse être modifiée.

2. Endoprothèse selon la revendication 1, caractérisée en ce que les moyens de retenue sont réalisés de façon à pouvoir pivoter à la manière d'un joint universel.

3. Endoprothèse selon la revendication 2, caractérisé en ce que les moyens de retenue sont constitués, du côté de la tige, par une saillie annulaire (6) à face inférieure sphérique convexe (7) et, du côté de la pièce d'appui sur l'os, par un creux (11) correspondant.

4. Endoprothèse selon l'une quelconque des revendications 1 à 3, caractérisée en ce que la pièce (9) d'appui sur l'os est réalisée en forme de fer à cheval et peut être emboîtée sur la tige par le côté.

# Fig.1

# Fig.2

# Fig. 3

# Fig.4

# Fig.5

# Fig.6